(19) Europäisches Patentamt
European Patent Office
Office européen des brevets

(11) Publication number: **0 516 034 A1**

(12) # EUROPEAN PATENT APPLICATION

(21) Application number: **92108832.4**

(51) Int. Cl.[5]: **A61K 31/66**

(22) Date of filing: **26.05.92**

(30) Priority: **28.05.91 IT MI911445**

(43) Date of publication of application:
**02.12.92 Bulletin 92/49**

(84) Designated Contracting States:
**AT BE CH DE DK ES FR GB GR IT LI LU MC NL PT SE**

(71) Applicant: **FIDIA S.p.A.**
**Via Ponte della Fabbrica 3-A**
**I-35031 Abano Terme (Padova)(IT)**

(72) Inventor: **Leon, Alberta**
**Piazza Napoli 19**
**I-35100 Padova(IT)**
Inventor: **Kirschner, Günter**
**Via Leonardo da Vinci, 14**
**I-35031 Abano terme (Padova)(IT)**

(74) Representative: **Gervasi, Gemma, Dr. et al**
**NOTARBARTOLO & GERVASI Srl 33, Viale Bianca Maria**
**I-20122 Milano(IT)**

(54) **Pharmaceutical compositions with immunosuppresive properties.**

(57) A new therapeutic use of phospholipid derivatives as immunosuppressants is described, these phospholipid derivatives having a general formula (I):

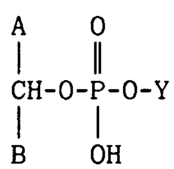

(I)

wherein

A = H; $-(CH_2)_nOH$, $-(CH_2)_n-O-CO-R$ (with n = 1, 2);

B =

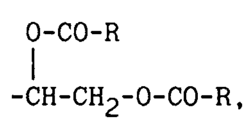

$-(CH_2)_m-O-CO-R$ (with m = 1,2,3,4)

with the limitation that when A = $-(CH_2)_n-O-CO-R$, m = 1,2;
and Y = serine or inositol, and wherein the R groups may be the same or different, and are radicals of acids that are aliphatic, aromatic, arylaliphatic, alicyclic, or heterocyclic on the condition that at least one R group is the radical of a monounsaturated or polyunsaturated fatty acid, and is preferably chosen from the group consisting of palmitoleic acid, oleic acid, linoleic acid, linolenic acid, and arachidonic acid.

EP 0 516 034 A1

FIELD OF THE INVENTION

The present invention refers to the use of phospholipid derivatives with a general formula (I):

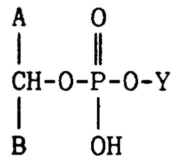

(I)

wherein

A = H; $-(CH_2)_nOH$, $-(CH_2)_n-O-CO-R$ (with n = 1, 2);

B =

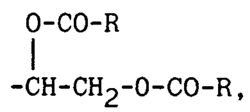

$-(CH_2)_m-O-CO-R$ (with m = 1,2,3,4)

with the limitation that when A = $-(CH_2)_n-O-CO-R$, m = 1,2;

and Y = serine or inositol, and wherein the R groups may be the same or different, and are radicals of acids that are aliphatic, aromatic, arylaliphatic, alicyclic, or heterocyclic on the condition that at least one R group is the radical of a monounsaturated or polyunsaturated fatty acid, and is preferably chosen from the group consisting of palmitoleic acid, oleic acid, linoleic acid, linoleneic acid, and arachidonic acid, for the preparation of pharmaceutical compositions with immunosuppressive properties.

**PRIOR ART**

It is known that phospholipids, and in particular phosphatidylserine (PS) and its derivatives, have various pharmacological properties, as was described, for example, in European patent application N° 90108222.2 registered in the name of the same Applicant and dated 30th of April 1990.

The various pharmacological properties of PS and its derivatives have always been exclusively related to the presence of the serine group, and no importance has been given to the fatty acids present in the glycerol radical.

It is also known from previous research carried out by the Applicant (D. Ponzin, C. Mancini, G. Toffano, A. Bruni, and G. Doria: 'Phosphatidylserine-Induced Modulation of the Immune Response in Mice - Effect of Intravenous Administration', Immunopharmacology 1989; 18: 167-176) that the intravenous administration of phosphatidylserine extracted from bovine brain tissue is capable of modulating the activity of the immune system in mice.

The extracted PS that was used for the purposes of this experiment, is actually a complex mixture of phosphatidylserines in which a large variety of saturated, monounsaturated, and polyunsaturated fatty acids are present. An analysis of its properties based on the present fatty acids has never been carried out.

In the above mentioned work, only the importance of the serine group was pointed out, and the same work also concluded that, as lysophosphatidylserine, also obtained by extraction, when administered under the same conditions showed similar properties to phosphatidylserine, the presence of unsaturated fatty acids in the structure of the molecule were of no importance in determining its effect on the immune system.

The effect on the immune system in mice shown in the aforementioned work by Ponzin et al., was, however, so limited that it was not possible to forecast any therapeutic use on humans.

Actually, until today the therapeutic use of phospholipid derivatives as immunosuppressants has never been proposed.

**Detailed Description**

Surprisingly, we have now found that phospholipid derivatives with a general formula (I):

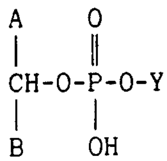 (I)

wherein

A = H; $-(CH_2)_nOH$, $-(CH_2)_n-O-CO-R$ (with n = 1, 2);

B =

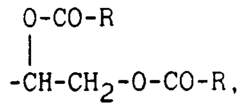

$-(CH_2)_m-O-CO-R$ (with m = 1,2,3,4)

with the limitation that when A = $-(CH_2)_n-O-CO-R$, m = 1,2;

and Y = serine or inositol, and wherein the R groups may be the same or different, and are radicals of acids that are aliphatic, aromatic, arylaliphatic, alicyclic, or heterocyclic, on the condition that at least one R group is the radical of an unsaturated fatty acid; act as immunosuppressants in the treatment of pathologies characterized by an altered immunological reactivity and/or symptoms of autoimmunity.

According to the present invention, the pharmacological activity of the compounds is clearly shown in the hereinafter reported data that follows, which particularly point out the critical structure/property relationship with reference to the presence of unsaturated fatty acid radicals in the tested phospolipid molecules.

In this research, the action of phospholipid derivatives on mononucleic cells from peripheral blood stimulated by phytohemaglutenin in order to start blastogenesis was analysed.

The results show that the proliferation of these cells (measured as DNA synthesis) is inhibited, in a non-cytotoxic way, by those molecules that possess a particular structure, and point out the existence of a structure/property relationship.

In particular, the molecular structure shows a pharmacological activity when the following characteristics are simultaneously satisfied:

1) A negatively charged polar head, preferably L-serine, which can be symetrically or assimetrically positioned with respect to the acyl chains;

2) At least one unsaturated acyl chain;

3) A phosphoril group.

The following abbreviations are used in the text:

| | |
|---|---|
| PS | - phosphatidylserine |
| PI | - phosphatidylinositol |
| PG | - phosphatidylglycerol |
| PC | - phosphatidylcolin |
| PA | - phosphatidic acid |
| PHA | - phytohemaglutenin |
| IL-2 | - interleukin 2 |
| PBMC | - peripheral blood mononucleic cells |
| PS-S1 | - 1,3-dipalmitoylglycero-2-phosphoryl-L-serine |
| PS-S10 | - 1,3-dioleylglycero-2-phosphoryl-L-serine |
| FCS | - foetal calf serum |

## METHODS AND MATERIALS

The following phospholipids were used: PS containing L-serine, obtained as per H. Eibel, 'Synthesis of Glycerophospholipids', Chemistry and Physics of Lipids, 26, 405-429, (1980), or single species of PS molecules extracted from bovine cortical tissue, separated using HPLC in an inverse phase as per G.M. Patton et al., 'Separation of Phospholipids and Individual Molecular Species of Phospholipids by High-Performance Liquid Chromatography', Journal of Lipid Research, 23, 190-196, (1982); PS-S1 lysin salt (Fidia); PS-S10 acid (Fidia); PS containing D-serine obtained from egg PC by transphosphatidylation in the presence of D-serine (supplied by Meito Sangyo Co., Tokyo); dipalmitoyl-PS, egg PC, PG derived from egg

PC, PA (Sigma); dimyristoyl-PS (Novabiochem), dioleyl-PS and PI (Avanti).

In particular, the PS-S1 and PS-S10 derivatives may be obtained according to the process described in European patent application N° 90108222.2 filed on the 30th of April 1990 in the name of the Applicant.

The purity of each phospholipid was checked by bi-dimensional TLC (L. Punzi, S. Todesco, G. Toffano, R. Catena, E. Bigon, and A. Bruni: 'Phospholipids in Inflammatory Synovial Effusions', International Rheumatology, 6, 7-11 (1986).

All the phospholipids were used in the form of small monolayer liposomes dispersed in a buffered saline solution (PBS), $Ca^{2+}$ free and containing 4 mM $Na_2KPO_4$, 150 mM NaCl, and 2.7 mM KCL (pH 7.4). The following procedure was followed: dried phospholipids were dissolved in an organic solvent, re-hydrated in PBS, and then the suspension was sonicated for 2 to 5 minutes in a bath. The exceptions to this standard preparation were: dipalmitoyl-PS, which was treated by means of a microprobe sound device for 2 minutes at room temperature, and salified PS-S1, which was directly dissolved in PBS and then sonicated.

Experimental System

The PBMCs were obtained by standard density gradient centrifugation of heparinized human venous blood through a Ficoll-Hypaque cushion. The cells were composed of 90% lymphocytes and 10% monocytes as assessed by acridine orange staining.

DNA Synthesis. The cells were re-suspended in RPMI + 5% FCS and spread on 3072 Falcon microtiter plates at a density of $10^5$ cells in 0.2 ml of final volume. They were cultured for 72 hours at 37°C with PHA 1 µg/ml in the presence or absence of phospholipids. The cells were marked with 1 uCi/well of [$^3$H]thymidine for the last 18 hours culture before being collected on filter paper and counted in a scintillation counter.

To exclude cytotoxic effect of used derivatives, cell viability (> 99%) was tested before, during, and after the incubation by the trypan blue exclusion test.

Flow Cytometry Analysis.

To confirm the DNA synthesis parameter data obtained, a second lymphocyte activity parameter was analysed - the expression of the IL-2 and transferrin receptors on the cell membrane.

To this purpose, after incubation with PHA 1 µg/ml for 72 hours in the presence or absence of PS, the cells were stained with fluorescent monoclonal antibodies against the above receptor, and then analysed on a FAC Star (Beckton-Dickinson) flow cytometer equipped with an argon ion laser operating at 480 nm and 200 mw.

**RESULTS**

Figure 1 shows the role of the phospholipid structure that was detected by analysing the action of PS type phospholipids and other phospholipids in inhibiting DNA synthesis in PBMC cultured for 72 hours with PHA 1 µg/ml, and shows representative data from 3 different experiments.

Figure 1A. (□) dimyristoyl PS; (▲) dipalmitoyl PS; (●) PS containing D-serine; (○) PS containing L-serine; (Δ) dioleyl PS.

Figure 1B. (Δ) PG; (○) PA; (●) PC; (▲) PI; average ± SEM of 4 to 13 experiments each carried out in triplicate with different donors.

Figure 1C. (○) PS-S1; (●) PS-S10.

The addition of PS to PHA activated PBMC results in a marked inhibition of DNA synthesis. 50% of the maximum effect is induced at 30 nmol PS/$10^6$ cells, corresponding to a concentration of 15 µM of PS in the medium. Cell viability was not affected by PS, as shown by the trypan blue exclusion test.

Dioleyl PS and PS-S10 showed the same activity as PS containing L-serine, whilst PS containing D-serine was less active (50% of maximum inhibition was observed at 60 nmol/$10^6$ cells).

On the contrary, dimyristoyl PS, dipalmitoyl PS and PS-S1 do not inhibit DNA synthesis, but slightly stimulate it. Of the other phospholipids, only PI is active.

Other negatively charged phospholipids, such as PA and PG, and isoelectric ones, such as PC, are totally inactive.

The results thus show that all active molecules must necessarily contain at least one unsaturated acyl chain and serine or inositol as a polar head. The most active derivatives are those with L-serine as a polar head. When this group is associated with glycerol, it can be indifferently symetrically or assymetrically positioned in respect of the acyl chain/s, as the results obtained with PS-S10 show.

The presence of PS also causes a strong reduction in the expression of the IL-2 and transferrin receptors induced by PHA in the PBMC - after 72 hours the PHA had activated the expression of the IL-2 and transferrin receptors in 77% and 54% of lymphocytes respectively, whilst in the presence of PS these percentages were reduced to 15% and 20% respectively.

In agreement with a close relationship between the inhibition of IL-2 receptor expression and the effects induced by PS and derivatives on DNA synthesis, it was observed that the addition of 10 U/ml of IL-2 to cells incubated with PHA does not remove the action of phospholipids.

The expression of the IL-2 and transferrin receptors is shown in figure 2. $10^5$ PBMC were cultured for 72 hours with PHA 1 µg/ml in the presence or absence of PS 60 nmol/$10^6$ cells. Fluorescence intensity (receptor expression) and light diffusion (cell size) were measured using flow cytometry. The data are reported as percentage of cells located in the shown region.

From the above data it can be concluded that only derivatives with particular minimum structural requisites have immunosuppressive properties. The study of the relationship between structure and activity shows that the immunosuppressive activity is present when the molecular structure is encompassing:

1) A negatively charged polar head, preferably L-serine, which can be symmetrically or asymmetrically positioned in respect of the acyl chain;

2) At least one unsaturated acyl chain;

3) A phosphoric group.

The phospholipid derivatives with a general formula (I) according to the present invention are active as immunosuppressants in the treatment of pathologies characterized by an altered immunological reactivity and/or related to autoimmunity, such as, for example, myasthenia gravis, Guillain-Barré, organ transplant pathologies (particularly kidney transplants), several types of collagen-vasculopathy (for example systemic lupus erythomatosus, necrotic vasculitis, sclerodermia, polymyositis, rheumatoid arthritis, and related pathologies such as Wegener's granulomatosis), regional enteritis, ulcerous colitis, chronic active hepatitis, glomerulonephritis, nephrosis syndrome, Goodpasture's syndrome, autoimmune hemolytic anaemia, idiopathic thrombocytopenia purpurea, pemphigus, allergy based pathologies (asthma, contact eczema), autoimmune endocrine illnesses (for example autoimmune thyroiditis, idiopathic subrenal atrophy, juvenile diabetes), interstitial pneumopathy, anaphylactic shock, and others.

The main active principles with a general formula (I) can be used, according to the present invention, to prepare pharmaceutical compositions which are effective in the above listed pathological situations, and in particular for the preparation of orally, parenterally or locally administered pharmaceutical compositions which contain a quantity of between 20 and 1000 mg of the active principle, and preferably between 40 and 350 mg, in association with usual pharmaceutical excipients.

For purely illustrative purposes, some examples of pharmaceutical compositions according to the invention are given hereinafter.

Example 1

A 2 ml phial contains:

| | |
|---|---|
| 1,3-dioleylglycero-2-phosphoryl-L-serine | 40.0 mg |
| Monobasic sodium phosphate | 2.14 mg |
| Dibasic sodium phosphate | 2.26 mg |
| Apyrogenous double-distilled water | 2 ml QS |

Example 2

A 3 ml phial contains:

| | |
|---|---|
| 1,3-dioleylglycero-2-phosphoryl-L-serine | 80.0 mg |
| Monobasic sodium phosphate | 3.21 mg |
| Dibasic sodium phosphate | 3.39 mg |
| Mannitol | 30 mg |
| Apyrogenous double-distilled water | 3 ml QS |

Example 3

A gelatine capsule contains:

| | |
|---|---|
| 1,3-dioleylglycero-2-phosphoryl-L-serine | 120 mg |
| Vegetable oil | 270 mg |
| Beeswax | 1 mg |

Example 4

A gelatine capsule contains:

| | |
|---|---|
| 1,3-dioleylglycero-2-phosphoryl-L-serine | 320 mg |
| Vegetable oil | 270 mg |
| Beeswax | 1 mg |

Example 5

A pill contains:

| | |
|---|---|
| 1,3-dioleylglycero-2-phosphoryl-L-serine | 60 mg |
| Mannitol | 100 mg |
| Micro-crystalline cellulose | 25 mg |
| Starch | 5 mg |
| Saccharose | 30 mg |
| Lacquer | 5 mg |

Example 6

An operculum contains:

| | |
|---|---|
| 1,3-dioleylglycero-2-phosphoryl-L-serine | 185 mg |
| Mannitol | 100 mg |
| Lactose | 100 mg |

**Claims**

**1.** The use of phospholipid derivatives with a general formula (I):

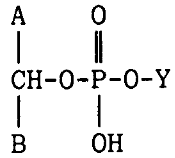

(I)

wherein

A = H; $-(CH_2)_nOH$, $-(CH_2)_n-O-CO-R$ (with n = 1, 2);

B =

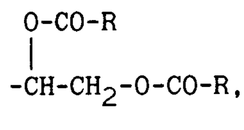

$-(CH_2)_m-O-CO-R$ (with m = 1,2,3,4)
with the limitation that when A = $-(CH_2)_n-O-CO-R$, m = 1,2;
and Y = serine or inositol, and wherein the R groups may be the same or different, and are radicals of acids that are aliphatic, aromatic, arylaliphatic, alicyclic, or heterocyclic on the condition that at least one R group is the radical of a monounsaturated or polyunsaturated fatty acid, or one of its pharmaceutically acceptable salts, for the preparation of pharmaceutical compositions with immunosuppressive properties.

2. Use as in Claim 1, characterized in that R is chosen from the group consisting of palmitoleic acid, oleic acid, linoleic acid, linolenic acid, and arachidonic acid.

3. Use as in Claim 1, characterized in that the phospholipid derivative is 1,3-dioleylglycero-2-phosphoryl-L-serine.

4. Use as in Claim 1, characterized in that the phospholipid derivative is 1-palmitoyl-2-oleylglycero-3-phosphoryl-L-serine.

5. Use as in Claim 1, characterized in that the compositions are active in the treatment of pathologies characterized by an altered immunological reactivity and/or related to autoimmunity, such as, Guillain-Barré, collagen-vasculopathies, organ transplant pathologies, glomerulonephritis, and autoimmune endocrine illnesses.

6. Use as in Claim 1, characterized in that the active principle content is between 20 and 1000 mg.

7. Use as in Claim 6, characterized in that the active principle content is between 40 and 350 mg.

8. Pharmaceutical compositions with immunosuppressive properties containing at least one compound with a general formula (I)

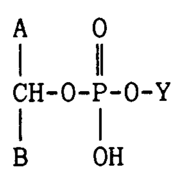
(I)

wherein
A = H; $-(CH_2)_nOH$, $-(CH_2)_n-O-CO-R$ (with n = 1, 2);
B =

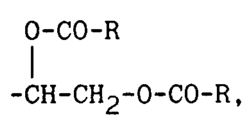

$-(CH_2)_m-O-CO-R$ (with m = 1,2,3,4)
with the limitation that when A = $-(CH_2)_n-O-CO-R$, m = 1,2;
and Y = serine or inositol, and wherein the R groups may be the same or different, and are radicals of acids that are aliphatic, aromatic, arylaliphatic, alicyclic, or heterocyclic on the condition that at least one R group is the radical of a monounsaturated or polyunsaturated fatty acid, or one of its pharmaceutically acceptable salts, in association with one or more pharmaceutically acceptable excipients.

**9.** Pharmaceutical compositions as in Claim 8, characterized in that R is chosen from the group consisting of palmitoleic acid, oleic acid, linoleic acid, linolenic acid, and arachidonic acid.

**10.** Pharmaceutical compositions as in Claim 8, characterized in that the phospholipid derivative is 1,3-dioleylglycero-2-phosphoryl-L-serine.

**11.** Pharmaceutical compositions as in Claim 8, characterized in that the phospholipid derivative is 1-palmitoyl-2-oleylglycero-3-phosphoryl-L-serine.

**12.** Pharmaceutical compositions as in Claim 8, characterized in fact that the compositions are active in the treatment of pathologies characterized by an altered immunological reactivity and/or related to autoimmunity, such as, Guillain-Barré, collagen-vasculopathies, organ transplant pathologies, glomerulonephritis, and autoimmune endocrine illnesses.

**13.** Pharmaceutical compositions as in Claim 8, characterized in that the active principle content is between 20 and 1000 mg.

**14.** Pharmaceutical compositions as in Claim 13, characterized in that the active principle content is between 40 and 350 mg.

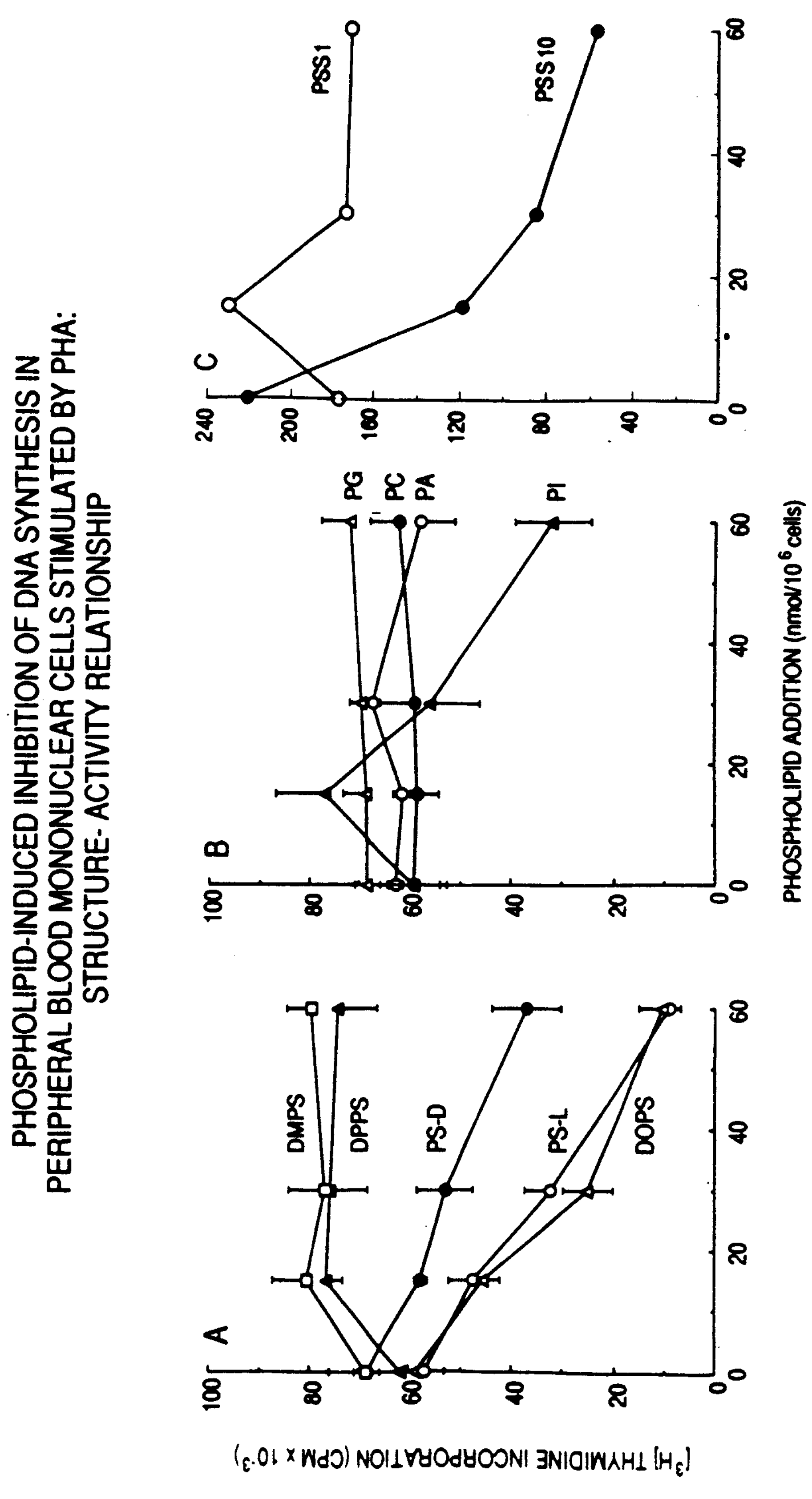

PHOSPHOLIPID-INDUCED INHIBITION OF DNA SYNTHESIS IN PERIPHERAL BLOOD MONONUCLEAR CELLS STIMULATED BY PHA: STRUCTURE- ACTIVITY RELATIONSHIP
A
B
C
DMPS
DPPS
PS-D
PS-L
DOPS
PG
PC
PA
PI
PSS1
PSS10
[3H] THYMIDINE INCORPORATION (CPM x 10-3)
PHOSPHOLIPID ADDITION (nmol/10 6 cells)


FIG. 1

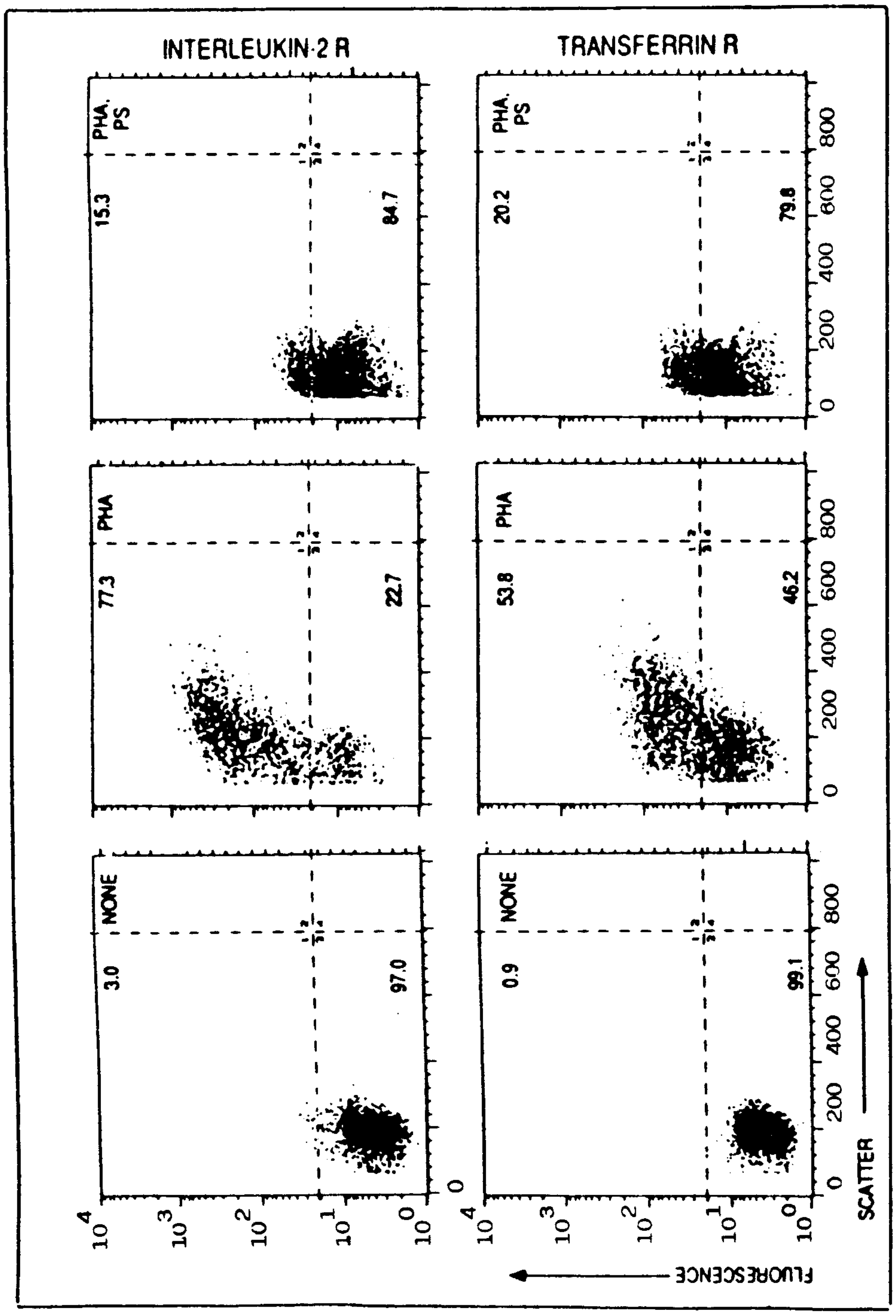
INTERLEUKIN-2 R
TRANSFERRIN R
NONE
PHA
PHA, PS
3.0
97.0
77.3
22.7
15.3
84.7
0.9
99.1
53.8
46.2
20.2
79.8
SCATTER
FLUORESCENCE
0
200
400
600
800
$10^0$
$10^1$
$10^2$
$10^3$
$10^4$

FIG. 2

European Patent Office

# EUROPEAN SEARCH REPORT

Application Number

EP 92 10 8832

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | THE JOURNAL OF BIOLOGICAL CHEMISTRY, vol. 264, no. 25, 5th September 1989, pages 14797-14805, The American Society for Biochemistry and Molecular Biology, Inc., US; M.-H. LEE et al.: "Phospholipid functional groups involved in protein kinase C activation, phorbol ester binding, and binding to mixed micelles" * Whole document * --- | 8-10,12 -14 | A 61 K 31/66 |
| X | WO-A-8 700 173 (CHEMIE LINZ AG) * Abstract; claims * --- | 8,9 | |
| X | WO-A-8 403 704 (PHARMACIA AB) * Abstract; claims * --- | 8,9 | |
| X | FR-A-2 649 322 (LABORATOIRES NATURA MEDICA) * Abstract; claims * --- | 8,9 | |
| D,A | IMMUNOPHARMACOLOGY, vol. 18, 1989, pages 167-176, Elsevier Science Publishers B.V. (Biomedical Division); D. PONZIN et al.: "Phosphatidylserine-induced modulation of the immune response in mice: effect of intravenous administration" * Whole document * --- | 1-14 | TECHNICAL FIELDS SEARCHED (Int. Cl.5) |
| D,A | EP-A-0 396 080 (FIDIA) * Abstract; page 2, lines 1-32; page 7, lines 19-27; claims * ----- | 1-14 | A 61 K |

The present search report has been drawn up for all claims

| Place of search | Date of completion of the search | Examiner |
|---|---|---|
| THE HAGUE | 09-07-1992 | GOETZ G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document